# EUROPEAN PATENT APPLICATION

(11) **EP 4 841 556 A1**
(43) Date of publication of application: **18.11.2026**
(21) Application number: 24917342.8
(22) Date of filing: 27.12.2024
(51) Int. Cl.: G06T 7/00, G06T 7/11

(54) **METHOD, DEVICE, AND COMPUTER PROGRAM FOR CLASSIFYING KERATITIS ACTIVITY USING ARTIFICIAL INTELLIGENCE**

(30) Priority: 10.01.2024 KR 20240004138
(71) Applicant: Samsung Life Public Welfare Foundation, Seoul 04348 (KR)
(72) Inventor: CHUNG, Myung Jin, Seoul 06351 (KR); YOO, Hakje, Seoul 06351 (KR); JUNG, Sung Ho, Seoul 06351 (KR); LIM, Dong Hui, Seoul 06351 (KR); WON, Yeo Kyoung, Seoul 06351 (KR)
(74) Representative: Plougmann Vingtoft a/s
(86) International application number: PCT/KR2024/021297
(87) International publication number: WO 2025/150748

(57) **Abstract**

The present invention is characterized in that a method for classifying keratitis activity using artificial intelligence comprises: an image receiving step of receiving an anterior segment image of a subject; a preprocessing step of acquiring an image of a pupil region from the received anterior segment image; and a training step of training an artificial intelligence model using the pupil region image labeled with a keratitis activity and a remission status.

## Description

### TECHNICAL FIELD

This work was supported by the Ministry of Science and ICT under Task Identification Number 1711180602 and Task Number 2021R1C1C1007795. The research management institution for the above task is the National Research Foundation of Korea, the research program name is "Individual Basic Research (Ministry of Science and ICT)", the research project name is "Development of Machine Learning-Based Cataract Clinical Diagnosis Technology and Treatment Algorithm According to Severity for National Eye Examination", the supervising institution is Samsung Life Public Welfare Foundation, and the research period is March 1, 2023 to February 29, 2024.

### BACKGROUND ART

Keratitis refers to a condition in which inflammation occurs in the cornea due to various causes. Because keratitis can cause perforation, vision deterioration, and vision loss depending on the pathogen and the condition of the patient, the appropriate treatment method, timing, and duration are important. In order to determine the appropriate treatment method, timing, and duration for keratitis, it is important to determine the keratitis activity.

The existing clinical criterion for determining keratitis activity is the result of a culture test of a pathogen collected from a lesion. However, such a method has a problem in that immediate determination and response are difficult because a certain amount of time is required until culture.

In this regard, Korean Registered Patent No. 10-2329313 discloses a corneal lesion analysis system and method using an anterior segment image, and a computer-readable recording medium. The prior art document is characterized in that it comprises an image acquisition unit that acquires an anterior segment image from the eye of a subject, and a feature extraction unit that extracts feature information regarding the lesion location and cause of the cornea from the anterior segment image by applying a convolution layer with the anterior segment image through machine learning based on a database storing clinical information that analyzes the lesion location and cause of the cornea of previously acquired subjects.

However, the prior art document has limitations in that, since the anterior segment image is directly input into the artificial intelligence without a preprocessing step to perform learning and extract features, a long time is required for computation, and only feature information regarding the lesion location and cause of the cornea is extracted while the keratitis activity cannot be extracted.

Accordingly, there is a demand for a method, apparatus, and computer program for classifying keratitis activity using artificial intelligence that can resolve the foregoing.

### DISCLOSURE OF INVENTION

### Technical Problem

It is an object of the present invention to provide a method, apparatus, and computer program for classifying keratitis activity using artificial intelligence that trains an artificial intelligence using a pupil region image extracted from an anterior segment image.

In addition, the present invention seeks to classify keratitis activity by inputting an image of a pupil region of a patient into a trained artificial intelligence model.

### Solution to Problem

In order to achieve the above object, the present invention is characterized in that a method for classifying keratitis activity using artificial intelligence comprises: an image receiving step of receiving an anterior segment image of a subject; a preprocessing step of acquiring an image of a pupil region from the received anterior segment image; and a training step of training an artificial intelligence model using the pupil region image labeled with a keratitis activity and a remission status.

Preferably, the preprocessing step may comprise: a location information extracting step of extracting pupil position information and pupil radius information from the anterior segment image; and a region extracting step of extracting a pupil region from the anterior segment image using the extracted pupil position information and radius information.

Preferably, the location information extracting step converts the anterior segment image to grayscale, uniformly converts a histogram distribution through normalization, and smoothly converts pixel-to-pixel value differences by applying a blur.

Preferably, the preprocessing step may group a plurality of pixels constituting the anterior segment image into units of a predetermined number, obtain a pixel average value for each group, and calculate a difference value of the pixel average values between adjacent groups.

Preferably, the preprocessing step may binarize the anterior segment image using a threshold value selected through an Otsu method based on a histogram of the calculated difference values of the pixel average values.

Preferably, the preprocessing step may emphasize boundaries by applying a contour to the binarized anterior segment image, and may pupil position information and pupil radius information using a Hough Circle Transform (CHT).

Preferably, the region extracting step may extract, in a circular shape, a range wider by a predetermined range than the extracted pupil radius, based on the extracted pupil position.

Preferably, the region extracting step may obtain a first region by inputting the extracted circular range into a prompt method of a Segment Anything Model (SAM), and may obtain a plurality of second regions by inputting the extracted circular range into an auto method of the Segment Anything Model (SAM).

Preferably, the region extracting step may compare the first region and the second regions using an Intersection over Union (IoU) method, extract the second region when a result is equal to or greater than a specific value, and extract the first region when a result is equal to or less than the specific value.

Preferably, the method may further comprise an activity classifying step of inputting an image of a pupil region of a subject into the trained artificial intelligence model and outputting a keratitis activity and a remission status.

The present invention is further characterized in that an apparatus for classifying keratitis activity using artificial intelligence comprises: a processor comprising one or more cores; and a memory, wherein the processor receives an anterior segment image of a subject, acquires an image of a pupil region from the received anterior segment image, trains an artificial intelligence model using the pupil region image labeled with a keratitis activity and a remission status, and inputs an image of a pupil region of a subject into the trained artificial intelligence model and outputs a keratitis activity and a remission status.

The present invention is further characterized in that a computer program stored on a computer-readable storage medium and comprising instructions that cause a computer to perform the following operations, the operations comprising: an image receiving operation of receiving an anterior segment image of a subject; a preprocessing operation of acquiring an image of a pupil region from the received anterior segment image; and a training operation of training an artificial intelligence model using the pupil region image labeled with a keratitis activity and a remission status, wherein the preprocessing operation comprises: a location information extracting operation of extracting pupil position information and pupil radius information from the anterior segment image; and a region extracting operation of extracting a pupil region from the anterior segment image using the extracted pupil position information and radius information.

### Advantageous Effects of Invention

The present invention has an advantage in that, by utilizing a pupil region image extracted from an anterior segment image for training, the inconvenience of existing manual work can be resolved and the training efficiency of the artificial intelligence model can be improved.

In addition, the present invention has an advantage in that it can provide information that a physician can reference by classifying the keratitis activity using only photographs taken during a camera examination that is commonly performed on keratitis patients.

In addition, the present invention has an advantage in that an appropriate response can be made at an early time through activity classification using artificial intelligence, instead of relying on culture test results that take a relatively long time.

### BRIEF DESCRIPTION OF DRAWINGS

The present invention has an advantage in that, by utilizing a pupil region image extracted from an anterior segment image for training, the inconvenience of existing manual work can be resolved and the training efficiency of the artificial intelligence model can be improved.

In addition, the present invention has an advantage in that it can provide information that a physician can reference by classifying the keratitis activity using only photographs taken during a camera examination that is commonly performed on keratitis patients.

In addition, the present invention has an advantage in that an appropriate response can be made at an early time through activity classification using artificial intelligence, instead of relying on culture test results that take a relatively long time.

### BEST MODE FOR CARRYING OUT THE INVENTION

A method for classifying keratitis activity using artificial intelligence, the method comprising: an image receiving step of receiving an anterior segment image of a subject; a preprocessing step of acquiring an image of a pupil region from the received anterior segment image; and a training step of training an artificial intelligence model using the pupil region image labeled with a keratitis activity and a remission status.

### MODE FOR CARRYING OUT THE INVENTION

Hereinafter, the present invention will be described in detail with reference to the contents set forth in the accompanying drawings. However, the present invention is not limited or restricted by the illustrative embodiments. Like reference numerals presented in each drawing indicate members that perform substantially the same function.

The objects and effects of the present invention may be naturally understood or made clearer by the following description, and the objects and effects of the present invention are not limited by the following description alone. In addition, in describing the present invention, if it is determined that a specific description of known technology related to the present invention may unnecessarily obscure the gist of the present invention, the detailed description thereof will be omitted.

The terms used in the present invention are used only to describe specific embodiments and are not intended to limit the present invention. Singular expressions include plural expressions unless the context clearly indicates otherwise. In the present application, terms such as "comprise" or "have" are intended to designate the existence of features, numbers, steps, operations, components, parts, or combinations thereof described in the description of the invention, and are to be understood as not precluding in advance the possibility of the existence or addition of one or more other features, numbers, steps, operations, components, parts, or combinations thereof.

Terms such as first and second may be used to describe various components, but the components should not be limited by the terms. The terms are used only for the purpose of distinguishing one component from another component. For example, a first component may be named a second component without departing from the scope of the present invention, and similarly a second component may also be named a first component.

Unless otherwise defined, all terms used herein, including technical or scientific terms, have the same meaning as commonly understood by a person of ordinary skill in the art to which the present invention pertains. Terms such as those defined in commonly used dictionaries are to be interpreted as having a meaning consistent with the meaning in the context of the related art, and are not to be interpreted in an ideal or excessively formal sense unless clearly defined in the present invention.

In interpreting components, it is to be interpreted as including a margin of error even if there is no separate explicit description. In the case of descriptions of temporal relationships, for example, when a temporal sequential relationship is described as "after," "following," "next," or "before," cases that are not consecutive are also included unless "immediately" or "directly" is used.

Hereinafter, the technical configuration of the present invention will be described in detail with reference to the accompanying drawings.

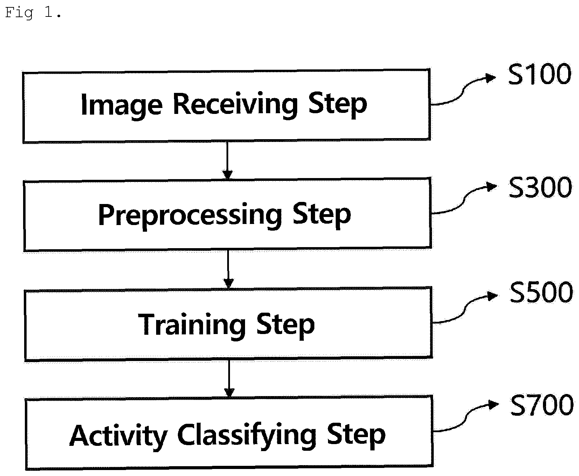

FIG. 1 illustrates a flowchart of a method for classifying keratitis activity using artificial intelligence according to an embodiment of the present invention. Referring to FIG. 1, the method for classifying keratitis activity using artificial intelligence may comprise an image receiving step (S100), a preprocessing step (S300), a training step (500), and an activity classifying step (S700).

An anterior segment image is a photograph taken of the eye area with a camera, and includes, in addition to the pupil which is the keratitis region, the eyelid, eyebrow, and the like, which constitute unnecessary information for training the model. In general, in order to remove such unnecessary information, a person manually processes the entire data, or mask data is created from a portion of the data to develop a segmentation model. The method for classifying keratitis activity using artificial intelligence can automatically extract the pupil region without such a process and utilize it for artificial intelligence training.

The method for classifying keratitis activity using artificial intelligence provides an algorithm that did not previously exist by appropriately combining fragmentary functions that have been previously proposed, such as the Otsu method and Hough Circle, or a large-scale artificial intelligence model such as a Segment Anything Medel (SAM). The method for classifying keratitis activity using artificial intelligence can identify and extract the position and region of the pupil even when the boundary between the iris and the conjunctiva is unclear due to disease progression or imaging technique. The method for classifying keratitis activity using artificial intelligence can classify the keratitis activity and whether remission has occurred for each pathogen.

The method for classifying keratitis activity using artificial intelligence can resolve the inconvenience of existing manual work and improve the training efficiency of the artificial intelligence model by utilizing a pupil region image extracted from an anterior segment image for training. In addition, the method for classifying keratitis activity using artificial intelligence has an advantage in that it can provide information that a physician can reference by classifying the keratitis activity using only photographs taken during a camera examination that is commonly performed on keratitis patients. In addition, the present invention can make an appropriate response at an early time through activity classification using artificial intelligence, instead of relying on culture test results that take a relatively long time.

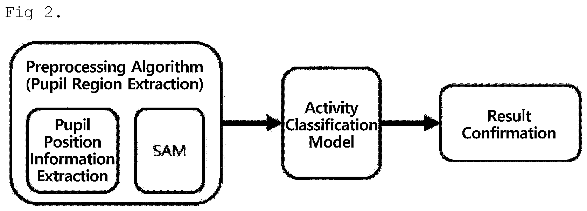

FIG. 2 illustrates a summary diagram of a method for classifying keratitis activity using artificial intelligence according to an embodiment of the present invention. Referring to FIG. 2, the method for classifying keratitis activity using artificial intelligence can acquire a pupil region image from the anterior segment image of a subject received in the image receiving step (S100) through the preprocessing step (S300), and input the acquired pupil region image into an artificial intelligence model.

The image receiving step (S100) can receive an anterior segment image of a subject. The anterior segment image can be acquired through a slit lamp microscope in a clinic, hospital, or the like, or through an image capture device mounted on a user device or the like. The user device may include any device that includes a capture module capable of capturing images, such as a smartphone, tablet, or digital camera.

The image receiving step (S100) can transmit the received anterior segment image to be preprocessed in the preprocessing step (S300), and can also cause the received anterior segment image to be input into an artificial intelligence model for training in the training step (S500).

The preprocessing step (S300) can acquire a pupil region image from the received anterior segment image. The preprocessing step (S300) can perform preprocessing in order to input the anterior segment image received in the image receiving step (S100) into an artificial intelligence model. The preprocessing step (S300) can provide the preprocessed image as training data for the artificial intelligence model. In addition, the preprocessing step (S300) can provide the preprocessed image to the trained artificial intelligence model in order to classify keratitis activity.

The anterior segment image is a photograph taken of the eye area and includes, in addition to the pupil which is the region where keratitis occurs, the eyelid, eyebrow, and the like. The preprocessing step (S300) according to an embodiment of the present invention can acquire only the pupil region image, which is the region where keratitis occurs, from the anterior segment image. In this manner, when an anterior segment image containing unnecessary information is used for training or classification of an artificial intelligence model, the model may make predictions based on information at a location other than the affected area (cornea). By removing the image surrounding the pupil, which constitutes unnecessary information, through the preprocessing step (S300) according to an embodiment of the present invention, the artificial intelligence model can accurately observe and learn the affected area (cornea), thereby improving the reliability and validity of the artificial intelligence model.

The preprocessing step (S300) may comprise a location information extracting step (S310) and a region extracting step (S330).

The location information extracting step (S310) can extract pupil position information and pupil radius information from the anterior segment image. The pupil position information and pupil radius information extracted in the location information extracting step (S310) can serve as a reference for the position and range of the region extracted in the region extracting step (S330). In addition, the pupil position information and pupil radius information extracted in the location information extracting step (S310) can be input into a Segment Anything Model (SAM) together with the anterior segment image in order to extract the pupil region.

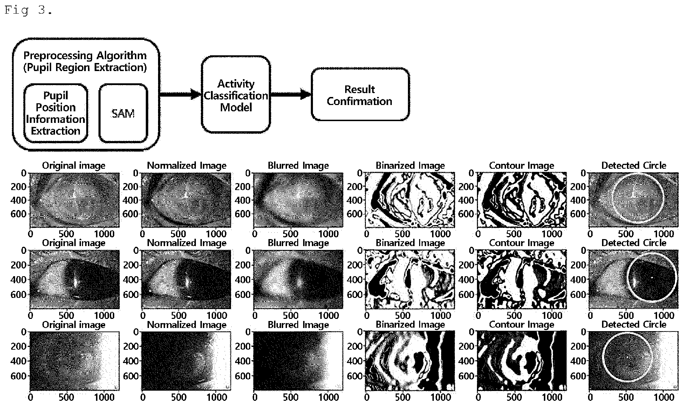

FIG. 3 illustrates a diagram for explaining the location information extracting step (S310) according to an embodiment of the present invention. With reference to FIG. 3, each function of the location information extracting step (S310) will be described.

The location information extracting step (S310) can convert the anterior segment image to grayscale, uniformly convert a histogram distribution through normalization, and smoothly convert pixel-to-pixel value differences by applying a blur. The leftmost photograph in FIG. 3 is the original anterior segment image, and the second photograph from the left is the anterior segment image converted to grayscale and normalized. The third photograph from the left is the photograph to which a blur has also been applied. The reason the location information extracting step (S310) performs such functions is to exclude unnecessary boundary portions caused by shadows, skin texture, blood vessels, fine hair, and the like, prior to extracting the pupil position information and pupil radius information.

The location information extracting step (S310) can group a plurality of pixels constituting the anterior segment image into units of a predetermined number, obtain a pixel average value for each group, and calculate a difference value of the pixel average values between adjacent groups. The location information extracting step (S310) can apply grayscale conversion, normalization, or blur, or the like, to the anterior segment image prior to performing this embodiment.

The location information extracting step (S310) can binarize the anterior segment image using a threshold value selected through an Otsu method based on a histogram of the calculated difference values of the pixel average values. The location information extracting step (S310) can obtain a clear boundary line from the anterior segment image through the function of grouping pixels, obtaining a pixel average value for each group, calculating a difference value of the pixel average values between adjacent groups, and binarization. The third photograph from the left in FIG. 3 is the photograph to which binarization has also been applied.

Here, the Otsu method refers to an algorithm that arbitrarily sets a boundary value to divide pixels into two classes, repeatedly obtains the intensity distribution of the two classes, and then selects the boundary value that makes the intensity distribution of the two classes most uniform. That is, the Otsu method may refer to obtaining an optimal threshold value at which the difference in the ratio of pixels binary-classified based on a specific threshold value is smallest.

The location information extracting step (S310) can emphasize boundaries by applying a contour to the binarized anterior segment image, and can extract pupil position information and pupil radius information using a Hough Circle Transform (CHT). Here, a contour refers to boundary line information of a region having the same color or the same pixel value. In addition, the Hough Circle Transform refers to a method of detecting circles using extracted edge information. The second photograph from the left in FIG. 3 is the photograph to which a contour has also been applied, and the rightmost photograph is the photograph to which the Hough Circle Transform has also been applied.

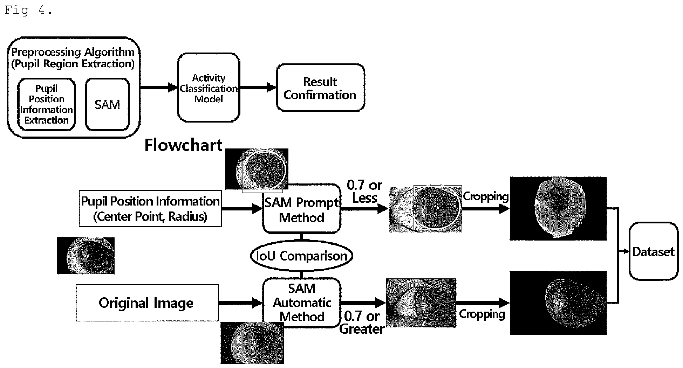

FIG. 4 illustrates a diagram for explaining the region extracting step (S330) according to an embodiment of the present invention. With reference to FIG. 4, each function of the region extracting step (S330) will be described.

The region extracting step (S330) can extract a pupil region from the anterior segment image using the extracted pupil position information and radius information.

The region extracting step (S330) can extract, in a circular shape, a range wider by a predetermined range than the extracted pupil radius, based on the extracted pupil position.

The region extracting step (S330) can obtain a first region by inputting the extracted circular range into a prompt method of a Segment Anything Model (SAM), and can obtain a plurality of second regions by inputting the extracted circular range into an auto method of the Segment Anything Model (SAM). In this case, when a plurality of second pupil regions are obtained by the auto method of the Segment Anything Model (SAM), all possible regions can be obtained, and among these, only regions of at least a specific area can be obtained. The first region obtained by the prompt method may be a pupil region, and the second regions obtained by the auto method of the SAM may be a plurality of pupil region candidates.

The region extracting step (S330) can compare the first region and the second regions using an Intersection over Union (IoU) method, extract the second region when a result is equal to or greater than a specific value, and extract the first region when a result is equal to or less than the specific value. Preferably, the specific value may be 0.7, but is not limited thereto.

The training step (500) can train an artificial intelligence model using the pupil region image labeled with a keratitis activity and a remission status. In this case, the pupil region image may refer to a dataset constructed in the region extracting step (S330).

The artificial intelligence model used in the embodiment of the present invention may be a deep neural network, and preferably may be a convolutional neural network (CNN).

A convolutional neural network (CNN) is a type of deep learning model for processing data having a grid pattern such as images, inspired by the organization of the visual cortex of animals. A convolutional neural network may generally comprise a convolutional layer, a pooling layer, and a fully connected layer. The convolutional layer and pooling layer may exist repeatedly within the neural network, and input data may be transformed into output through these layer hierarchies. The convolutional layer uses a kernel (or mask) for feature extraction, wherein an element-wise product between each element of the kernel and the input value is calculated and summed at each position to obtain an output value, which is referred to as a feature map. This procedure can be repeated by applying multiple kernels to form any number of feature maps. In a convolutional neural network, the convolutional and pooling layers perform feature extraction, while the fully connected layer maps the extracted features to a final output such as a classification operation.

A convolutional neural network and other such neural networks can be trained in a direction that minimizes output errors. Separately from the forward propagation process of extracting values from the input layer to the output layer, backpropagation occurs within the neural network, which calculates the error between the input training data and the output value of the neural network for the training data, and updates the connection weights of the nodes of each layer in order to reduce such errors. The training process in a convolutional neural network can be summarized as a process of finding a kernel that extracts an output value with the least error based on given training data. The kernel is the only parameter that is automatically learned during the training process of the convolutional layer. On the other hand, in a convolutional neural network, the kernel size, number of kernels, padding, and the like are hyperparameters that must be set before the training process begins, and accordingly, different convolutional neural network models can be distinguished according to the kernel size, number of kernels, and the number of convolutional layers and pooling layers.

A neural network can be trained in at least one of the following manners: supervised learning, which uses training data in which a correct answer is labeled for each piece of training data; unsupervised learning, in which correct answers are not labeled in the training data; semisupervised learning; or reinforcement learning. In this case, an error can be calculated by comparing the output through the neural network with a label or training data, and the calculated error is backpropagated in a reverse direction within the neural network (i.e., from the output layer toward the input layer), and the connection weight of each node of each layer of the neural network can be updated according to the backpropagation. The amount of change in the connection weight of each updated node can be determined according to a learning rate.

Overfitting is a phenomenon in which errors increase even as the number of training instances increases, as training on training data occurs excessively in the neural network. Overfitting can act as a cause of increasing errors in machine learning algorithms, and various optimization methods can be used to prevent such overfitting. In order to prevent overfitting, methods such as increasing training data, regularization, dropout that deactivates some nodes of the network during the training process, and utilization of a batch normalization layer can be applied.

The activity classifying step (S700) can input an image of a pupil region of a subject into the trained artificial intelligence model and output a keratitis activity and a remission status.

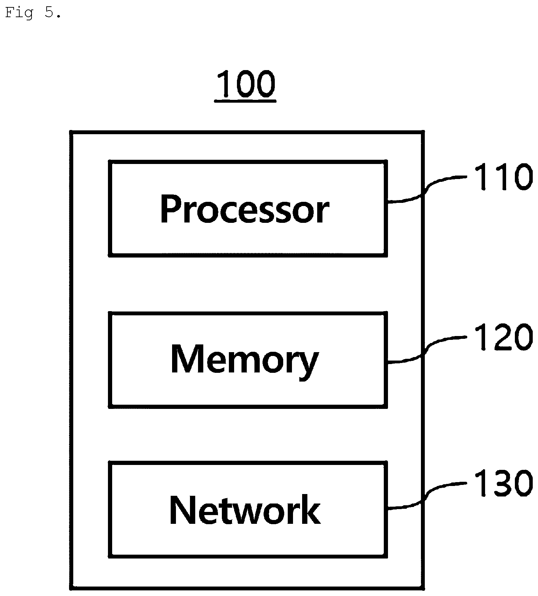

FIG. 5 illustrates a configuration diagram of an apparatus 100 for classifying keratitis activity using artificial intelligence according to an embodiment of the present invention. Referring to FIG. 5, the illustrated configuration of the apparatus 100 for classifying keratitis activity using artificial intelligence is merely a simplified example. In one embodiment of the present invention, the apparatus 100 for classifying keratitis activity using artificial intelligence may comprise other configurations for performing the computing environment of the apparatus 100, and only some of the disclosed configurations may constitute the apparatus 100.

The apparatus 100 for classifying keratitis activity using artificial intelligence may comprise a processor 110 comprising one or more cores, a memory 120, and a network 130.

The processor 110 may comprise one or more cores, and may comprise a processor for data analysis and deep learning, such as a central processing unit (CPU), a general purpose graphics processing unit (GPGPU), or a tensor processing unit (TPU) of a computing device. The processor 110 can read a computer program stored in the memory 120 and perform data processing for machine learning according to one embodiment of the present disclosure. According to one embodiment of the present disclosure, the processor 110 can perform operations for training a neural network. The processor 110 can perform calculations for training a neural network, such as processing input data for training in deep learning (DL), extracting features from input data, calculating errors, and updating weights of the neural network using backpropagation. At least one of the CPU, GPGPU, and TPU of the processor 110 can process training of a network function. For example, the CPU and the GPGPU together can process training of a network function and data classification using the network function. In addition, in one embodiment of the present disclosure, processors of a plurality of computing devices can be used together to process training of a network function and data classification using the network function. In addition, a computer program executed in a computing device according to one embodiment of the present disclosure may be a CPU-, GPGPU-, or TPU-executable program.

The processor 110 can receive an anterior segment image of a subject. The processor 110 can perform the aforementioned image receiving step (S100).

The processor 110 can acquire an image of a pupil region from the received anterior segment image. The processor 110 can perform the aforementioned preprocessing step (S300).

The processor 110 can train an artificial intelligence model using the pupil region image labeled with a keratitis activity and a remission status. The processor 110 can perform the aforementioned training step (S300).

The processor 110 can input an image of a pupil region of a subject into the trained artificial intelligence model and output a keratitis activity and a remission status. The processor 110 can perform the aforementioned activity classifying step (S700).

The memory 120 can store any form of information generated or determined by the processor 110 and any form of information received by the network 130.

The memory 120 may comprise at least one type of storage medium among a flash memory type, a hard disk type, a multimedia card micro type, a card-type memory (for example, SD or XD memory), random access memory (RAM), static random access memory (SRAM), read-only memory (ROM), electrically erasable programmable read-only memory (EEPROM), programmable read-only memory (PROM), magnetic memory, a magnetic disk, and an optical disk. The computing device 100 may also operate in association with web storage that performs the storage function of the memory 120 on the Internet. The foregoing description of the memory is merely exemplary, and the present disclosure is not limited thereto.

The network 130 can use any form of known wired or wireless communication system. The network 130 can receive endoscope images and the like from related devices or related systems.

The network 130 can transmit and receive information processed by the processor 110, user interfaces, and the like through communication with other terminals. For example, the network 130 can provide a user interface generated by the processor 100 to a client (e.g., a user terminal). In addition, the network 130 can receive an external input of a user authorized as a client and transmit it to the processor 110. In this case, the processor 110 can process operations such as outputting, modifying, changing, and adding information provided through the user interface based on the external input of the user received from the network 130.

Meanwhile, the apparatus 100 for classifying keratitis activity using artificial intelligence according to one embodiment of the present disclosure may comprise a server as a computing system that transmits and receives information through communication with a client. In this case, the client may be any form of terminal capable of accessing the server.

In an additional embodiment, the apparatus 100 for classifying keratitis activity using artificial intelligence may also comprise any form of terminal that receives data resources generated by an arbitrary server and performs additional information processing.

A computer program for classifying keratitis activity using artificial intelligence, which is another embodiment of the present invention, can perform an image receiving operation, a preprocessing operation, a training operation, and an activity classifying operation. The computer program for classifying keratitis activity using artificial intelligence may comprise instructions stored on a computer-readable storage medium that cause a computer to perform the following operations.

The image receiving operation can receive an anterior segment image of a subject. The image receiving operation refers to the operation performed in the aforementioned image receiving step (S100).

The preprocessing operation can acquire an image of a pupil region from the received anterior segment image. The preprocessing operation may comprise a location information extracting operation and a region extracting operation. The preprocessing operation refers to the operation performed in the aforementioned preprocessing step (S300).

The location information extracting operation can extract pupil position information and pupil radius information from the anterior segment image. The location information extracting operation refers to the operation performed in the aforementioned location information extracting step (S310).

The region extracting operation can extract a pupil region from the anterior segment image using the extracted pupil position information and radius information. The region extracting operation refers to the operation performed in the aforementioned region extracting step (S330).

The training operation can train an artificial intelligence model using the pupil region image labeled with a keratitis activity and a remission status. The training operation refers to the operation performed in the aforementioned training step (S500).

The activity classifying operation can input an image of a pupil region of a subject into the trained artificial intelligence model and output a keratitis activity and a remission status. The activity classifying operation refers to the operation performed in the aforementioned activity classifying step (S700).

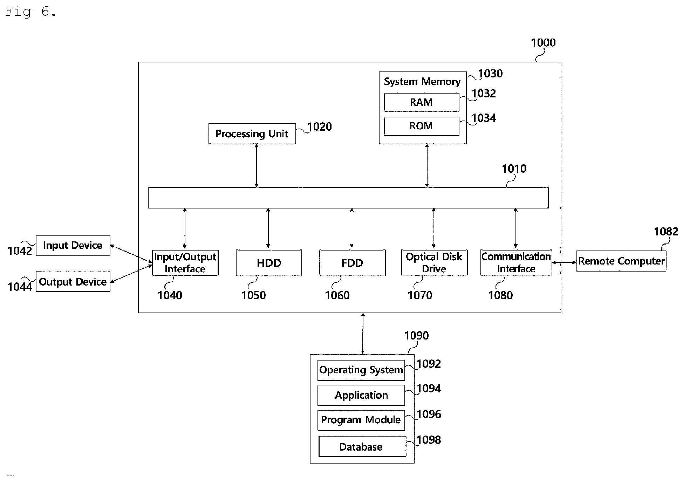

FIG. 6 illustrates a schematic diagram of a computing environment according to an embodiment of the present invention.

Although the present disclosure has been described above as being generally implementable by a computing device, a person skilled in the art will appreciate that the present disclosure can be implemented in combination with computer-executable instructions and/or other program modules executable on one or more computers, and/or as a combination of hardware and software.

In general, program modules comprise routines, programs, components, data structures, and the like that perform particular tasks or implement particular abstract data types. In addition, a person skilled in the art will appreciate that the method of the present disclosure can be practiced with other computer system configurations, including single-processor or multiprocessor computer systems, minicomputers, mainframe computers, as well as personal computers, handheld computing devices, microprocessor-based or programmable consumer electronics, and the like, each of which can be connected and operate with one or more associated devices.

The described embodiments of the present disclosure can also be practiced in distributed computing environments where certain tasks are performed by remote processing devices that are connected through a communication network. In a distributed computing environment, program modules can be located in both local and remote memory storage devices.

A computer typically comprises a variety of computer-readable media. Any medium accessible by a computer can be a computer-readable medium, and such computer-readable media comprise volatile and non-volatile media, transitory and non-transitory media, and removable and non-removable media. By way of example, and not limitation, computer-readable media may comprise computer-readable storage media and computer-readable transmission media. Computer-readable storage media comprise volatile and non-volatile media, transitory and non-transitory media, and removable and non-removable media implemented in any method or technology for storage of information such as computer-readable instructions, data structures, program modules, or other data. Computer-readable storage media comprise, but are not limited to, RAM, ROM, EEPROM, flash memory or other memory technology, CD-ROM, digital video disk (DVD) or other optical disk storage devices, magnetic cassettes, magnetic tapes, magnetic disk storage devices or other magnetic storage devices, or any other medium that can be accessed by a computer and used to store desired information.

A computer-readable transmission medium typically embodies computer-readable instructions, data structures, program modules, or other data in a modulated data signal such as a carrier wave or other transport mechanism, and comprises all information delivery media. The term modulated data signal means a signal that has one or more of its characteristics set or changed so as to encode information in the signal. By way of example, and not limitation, computer-readable transmission media comprise wired media such as a wired network or direct-wired connection, and wireless media such as acoustic, RF, infrared, and other wireless media. Combinations of any of the above-described media are also to be included within the scope of computer-readable transmission media.

An exemplary environment implementing various aspects of the present disclosure comprising a computer 1000 is illustrated, wherein the computer 1000 comprises a processing unit 1020, a system memory 1030, and a system bus 1010. The system bus 1010 connects system components, including but not limited to the system memory 1030, to the processing unit 1020. The processing unit 1020 can be any of various commercially available processors. Dual processor and other multiprocessor architectures can also be used as the processing unit 1020.

The system bus 1010 can be any of several types of bus structures that can additionally be interconnected to a memory bus, a peripheral bus, and a local bus using any of a variety of commercially available bus architectures. The system memory 1030 comprises read-only memory (ROM) 1034 and random access memory (RAM) 1032. A basic input/output system (BIOS) is stored in non-volatile memory 1034 such as ROM, EPROM, and EEPROM, and this BIOS comprises basic routines that help transfer information between components within the computer 1000, such as during startup. The RAM 1032 may also comprise high-speed RAM such as static RAM for caching data.

The computer 1000 also comprises an internal hard disk drive (HDD) 1050 (e.g., EIDE, SATA) - which internal hard disk drive 1050 can also be configured for external use within a suitable chassis (not shown) - a magnetic floppy disk drive (FDD) 1060 (e.g., for reading from or writing to a removable diskette), and an optical disk drive 1070 (e.g., for reading a CD-ROM disk or reading from or writing to other high-capacity optical media such as DVD). The hard disk drive 1050, magnetic disk drive 1060, and optical disk drive 1070 can be connected to the system bus 1010 by a hard disk drive interface, a magnetic disk drive interface, and an optical drive interface, respectively. The interface for external drive implementations comprises at least one or both of Universal Serial Bus (USB) and IEEE 1394 interface technologies.

These drives and their associated computer-readable media provide non-volatile storage of data, data structures, computer-executable instructions, and the like. In the case of the computer 1000, the drives and media correspond to storing any data in a suitable digital format. Although the description of computer-readable media above refers to an HDD, a removable magnetic disk, and removable optical media such as CD or DVD, a person skilled in the art will appreciate that other types of media readable by a computer, such as zip drives, magnetic cassettes, flash memory cards, cartridges, and the like, can also be used in the exemplary operating environment, and further that any such media can comprise computer-executable instructions for performing the methods of the present disclosure.

A number of program modules can be stored in the drives and RAM 1032, comprising an operating system 1092, one or more application programs 1094, other program modules 1096, and a database 1098. All or portions of the operating system, applications, modules, and/or data can also be cached in the RAM 1032. It will be appreciated that the present disclosure can be implemented in various commercially available operating systems or combinations of operating systems.

A user can enter commands and information into the computer 1000 through one or more wired/wireless input devices 1042, for example, a pointing device such as a keyboard and mouse. Other input devices (not shown) may comprise a microphone, IR remote control, joystick, game pad, stylus pen, touch screen, and the like. These and other input devices are often connected to the processing unit 1020 through an input/output interface 1040 connected to the system bus 1010, but can be connected by other interfaces such as a parallel port, IEEE 1394 serial port, game port, USB port, IR interface, and the like.

A monitor or other type of display device is also connected to the system bus 1010 through an interface such as a video adapter. In addition to a monitor, a computer typically comprises other peripheral output devices (not shown) such as speakers, a printer, and the like.

The computer 1000 can operate in a networked environment using logical connections to one or more remote computers, such as remote computer(s) 1082, through wired and/or wireless communications. The remote computer(s) 1082 can be a workstation, a computing device computer, a router, a personal computer, a portable computer, a microprocessor-based entertainment device, a peer device, or other common network node, and typically comprises many or all of the components described with respect to the computer 1000. The logical connections comprise wired/wireless connections to a local area network (LAN) and/or a larger network, for example, a wide area network (WAN). Such LAN and WAN networking environments are commonplace in offices and companies, and facilitate enterprise-wide computer networks such as intranets, all of which can be connected to a worldwide computer network, for example, the Internet.

When used in a LAN networking environment, the computer 1000 is connected to a local network (not shown) through a wired and/or wireless communication network interface or adapter (not shown). The adapter (not shown) can facilitate wired or wireless communication to the LAN (not shown), which LAN (not shown) also comprises a wireless access point installed therein for communicating with a wireless adapter (not shown). When used in a WAN networking environment, the computer 1000 can comprise a modem (not shown), or is connected to a communication computing device on the WAN (not shown), or has other means for establishing communication over the WAN (not shown), such as through the Internet. The modem (not shown), which can be an internal or external and wired or wireless device, is connected to the system bus 1010 through a serial port interface (not shown). In a networked environment, program modules described with respect to the computer 1000, or portions thereof, can be stored in a remote memory/storage device (not shown). It will be appreciated that the network connections shown are exemplary and other means of establishing a communications link between the computers can be used.

The computer 1000 operates to communicate with any wireless devices or entities deployed and operating in wireless communication, for example, a printer, scanner, desktop and/or portable computer, portable data assistant (PDA), communication satellite, any equipment or location associated with a wirelessly detectable tag, and a telephone. This comprises at least Wi-Fi and Bluetooth wireless technologies. Thus, the communication can be a predefined structure as with a conventional network, or simply an ad hoc communication between at least two devices.

Wi-Fi (Wireless Fidelity) enables connection to the Internet and the like without wires. Wi-Fi is a wireless technology, like cell phones, that enables such devices, for example, computers, to transmit and receive data indoors and outdoors, i.e., anywhere within the coverage area of a base station. Wi-Fi networks use a wireless technology called IEEE 802.11 (a, b, g, etc.) to provide secure, reliable, and high-speed wireless connections. Wi-Fi can be used to connect computers to each other, to the Internet, and to wired networks (using IEEE 802.3 or Ethernet). Wi-Fi networks can operate in unlicensed 2.4 and 5 GHz radio bands, for example, at 11 Mbps (802.11a) or 54 Mbps (802.11b) data rates, or in products comprising both bands (dual band).

A person of ordinary skill in the art of the present disclosure will understand that information and signals can be represented using any of a variety of different technologies and techniques. For example, data, instructions, commands, information, signals, bits, symbols, and chips that may be referenced throughout the above description can be represented by voltages, currents, electromagnetic waves, magnetic fields or particles, optical fields or particles, or any combination thereof.

A person of ordinary skill in the art of the present disclosure will understand that the various illustrative logical blocks, modules, processors, means, circuits, and model steps described in connection with the embodiments disclosed herein can be implemented by electronic hardware, various forms of program or design code (referred to herein as software, for convenience), or a combination of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. A person of ordinary skill in the art of the present disclosure can implement the described functionality in varying ways for each particular application, but such implementation decisions should not be interpreted as causing a departure from the scope of the present disclosure.

The various embodiments described herein can be implemented as a method, apparatus, or article of manufacture using standard programming and/or engineering techniques. The term article of manufacture comprises a computer program, carrier, or media accessible from any computer-readable storage device. For example, computer-readable storage media comprise, but are not limited to, magnetic storage devices (e.g., hard disk, floppy disk, magnetic strip, etc.), optical disks (e.g., CD, DVD, etc.), smart cards, and flash memory devices (e.g., EEPROM, card, stick, key drive, etc.). In addition, the various storage media presented herein comprise one or more devices and/or other machine-readable media for storing information.

It is to be understood that the specific order or hierarchy of steps in the processes presented is an example of illustrative approaches. It is to be understood that the specific order or hierarchy of steps in the processes can be rearranged within the scope of the present disclosure based on design priorities. The appended method claims provide elements of the various steps in a sample order but are not meant to be limited to the specific order or hierarchy presented.

The description of the presented embodiments is provided to enable any person of ordinary skill in the art of the present disclosure to use or practice the present disclosure. Various modifications to these embodiments will be apparent to a person of ordinary skill in the art of the present disclosure, and the general principles defined herein can be applied to other embodiments without departing from the scope of the present disclosure. Thus, the present disclosure is not intended to be limited to the embodiments presented herein, but is to be accorded the widest scope consistent with the principles and novel features presented herein.

The embodiments of the present invention described above are not implemented only through a device and method, but may also be implemented through a program that realizes functions corresponding to the configuration of the embodiments of the present invention or a recording medium on which the program is recorded. Such a recording medium can be executed not only on a server but also on a user terminal.

Although the embodiments of the present invention have been described in detail above, the scope of rights of the present invention is not limited thereto, and various modifications and improvements made by a person skilled in the art using the basic concept of the present invention as defined in the following claims also fall within the scope of rights of the present invention.

Although the present invention has been described in detail through representative embodiments above, a person of ordinary skill in the art to which the present invention pertains will understand that various modifications are possible with respect to the above-described embodiments within the limits that do not depart from the scope of the present invention. Therefore, the scope of rights of the present invention should not be limited to the described embodiments, but should be determined by all changes or modified forms derived from the claims set forth hereinafter as well as the concept of equivalents of the claims.

### Industrial Applicability

It is an object of the present invention to provide a method, apparatus, and computer program for classifying keratitis activity using artificial intelligence that trains an artificial intelligence using a pupil region image extracted from an anterior segment image.

In addition, the present invention seeks to classify keratitis activity by inputting an image of a pupil region of a patient into a trained artificial intelligence model.

## Claims

1. A method for classifying keratitis activity using artificial intelligence, the method comprising:
an image receiving step of receiving an anterior segment image of a subject;
a preprocessing step of acquiring an image of a pupil region from the received anterior segment image; and
a training step of training an artificial intelligence model using the pupil region image labeled with a keratitis activity and a remission status.

2. The method of claim 1, wherein the preprocessing step comprises:
a location information extracting step of extracting pupil position information and pupil radius information from the anterior segment image; and
a region extracting step of extracting a pupil region from the anterior segment image using the extracted pupil position information and radius information

3. The method of claim 2, wherein the location information extracting step comprises:
converting the anterior segment image to grayscale, uniformly converting a histogram distribution through normalization, and smoothly converting pixel-to-pixel value differences by applying a blur.

4. The method of claim 2, wherein the location information extracting step comprises:
grouping a plurality of pixels constituting the anterior segment image into units of a predetermined number, obtaining a pixel average value for each group, and calculating a difference value of the pixel average values between adjacent groups.

5. The method of claim 4, wherein the location information extracting step comprises:
binarizing the anterior segment image using a threshold value selected through an Otsu method based on a histogram of the calculated difference values of the pixel average values.

6. The method of claim 5, wherein the location information extracting step comprises:
emphasizing boundaries by applying a contour to the binarized anterior segment image, and extracting pupil position information and pupil radius information using a Hough Circle Transform (CHT).

7. The method of claim 2, wherein the region extracting step comprises:
extracting, in a circular shape, a range wider by a predetermined range than the extracted pupil radius, based on the extracted pupil position.

8. The method of claim 7, wherein the region extracting step comprises:
obtaining a first region by inputting the extracted circular range into a prompt method of a Segment Anything Model (SAM); and
obtaining a plurality of second regions by inputting the extracted circular range into an auto method of the Segment Anything Model (SAM).

9. The method of claim 8, wherein the region extracting step comprises:
comparing the first region and the second regions using an Intersection over Union (IoU) method, extracting the second regions when a result is equal to or greater than a specific value, and extracting the first region when the result is equal to or less than the specific value.

10. The method of claim 1, further comprising:
an activity classifying step of inputting an image of a pupil region of a subject into the trained artificial intelligence model and outputting a keratitis activity and a remission status.

11. An apparatus for classifying keratitis activity using artificial intelligence, the apparatus comprising:
a processor comprising one or more cores; and
a memory,
wherein the processor is configured to:
receive an anterior segment image of a subject;
acquire an image of a pupil region from the received anterior segment image;
train an artificial intelligence model using the pupil region image labeled with a keratitis activity and a remission status; and
input an image of a pupil region of a subject into the trained artificial intelligence model and output a keratitis activity and a remission status.

12. A computer program stored on a computer-readable storage medium and comprising instructions that cause a computer to perform the following operations, the operations comprising:
an image receiving operation of receiving an anterior segment image of a subject;
a preprocessing operation of acquiring an image of a pupil region from the received anterior segment image; and
a training operation of training an artificial intelligence model using the pupil region image labeled with a keratitis activity and a remission status,
wherein the preprocessing operation comprises:
a location information extracting operation of extracting pupil position information and pupil radius information from the anterior segment image; and a region extracting operation of extracting a pupil region from the anterior segment image using the extracted pupil position information and radius information.
